# EUROPEAN PATENT SPECIFICATION

(11) **EP 1 279 912 B1**
(45) Date of publication and mention of the grant of the patent: **02.03.2005**
(21) Application number: 02022943.1
(22) Date of filing: 11.12.1998
(51) Int. Cl.: F25J 1/00, A61K 49/00, G01R 33/28

(54) **Methods of collecting and thawing polarised gases, associated accumulators and heating jackets**
Verfahren zum Sammeln und Auftauen polarisierter Gase, dessen Sammler und Heizmanschette
Méthode de récupération et de décongélation de gaz polarisés, récupérateur associé et chemise de chauffage

(30) Priority: 12.12.1997 US 69435 P
(43) Date of publication of application: 29.01.2003
(62) Divisional of application: 98967030.2
(73) Proprietor: Medi-Physics, Inc., Princeton, NJ 08540 (US)
(72) Inventor: Deaton, Daniel, Apex, NC 27502 (US); Driehuys, Bastiaan, Chapel Hill, NC 27515 (US); Hasson, Kenton C., Charlottesville, VA 22901 (US); Zollinger, David, Belmont, MA 02478 (US); Langhorn, Alan, Solana Beach, CA 92075 (US)
(74) Representative: Canning, Lewis R.

(56) References cited:
- US-A- 5 545 396
- US-A- 5 642 625

## Description

### Field of the Invention

The present invention relates to a cryogenic accumulator and a method for collecting frozen polarized noble gases.

### Background of the Invention

Conventionally, MRI has been used to produce images by exciting the nuclei of hydrogen molecules (present in water protons) in the human body. However, it has recently been discovered that polarized noble gases can produce improved images of certain areas and regions of the body which have heretofore produced less than satisfactory images in this modality. Polarized Helium 3 ("³He") and Xenon-129 ("¹²⁹Xe") have been found to be particularly suited for this purpose. Unfortunately, as will be discussed further below, the polarized state of the gases is sensitive to handling and environmental conditions and can, undesirably, decay from the polarized state relatively quickly.

Hyperpolarizers are used to produce and accumulate polarized noble gases. Hyperpolarizers artificially enhance the polarization of certain noble gas nuclei (such as ¹²⁹Xe or ³He) over the natural or equilibrium levels, *i.e.,* the Boltzmann polarization. Such an increase is desirable because it enhances and increases the Magnetic Resonance Imaging ("MRI") signal intensity, allowing physicians to obtain better images of the substance in the body. *See* U. S. Patent No. 5,545,396 to Albert et al..

In order to produce the hyperpolarized gas, the noble gas is typically blended with optically pumped alkali metal vapors such as rubidium ("Rb"). These optically pumped metal vapors collide with the nuclei of the noble gas and hyperpolarize the noble gas through a phenomenon known as "spin-exchange". The "optical pumping" of the alkali metal vapor is produced by irradiating the alkali-metal vapor with circularly polarized light at the wavelength of the first principal resonance for the alkali metal *(e.g.,* 795 nm for Rb). Generally stated, the ground state atoms become excited, then subsequently decay back to the ground state. Under a modest magnetic field (10 Gauss), the cycling of atoms between the ground and excited states can yield nearly 100% polarization of the atoms in a few microseconds. This polarization is generally carried by the lone valence electron characteristics of the alkali metal. In the presence of non-zero nuclear spin noble gases, the alkali-metal vapor atoms can collide with the noble gas atoms in a manner in which the polarization of the valence electrons is transferred to the noble-gas nuclei through a mutual spin flip "spin-exchange".

Conventionally, lasers have been used to optically pump the alkali metals. Various lasers emit light signals over various wavelength bands. In order to improve the optical pumping process for certain types of lasers (particularly those with broader bandwidth emissions), the absorption or resonance line width of the alkali metal can be made broader to more closely correspond with the particular laser emission bandwidth of the selected laser. This broadening can be achieved by pressure broadening, *i.e.,* by using a buffer gas in the optical pumping chamber. Collisions of the alkali metal vapor with a buffer gas will lead to a broadening of the alkali's absorption bandwidth.

For example, it is known that the amount of polarized ¹²⁹Xe which can be produced per unit time is directly proportional to the light power absorbed by the Rb vapor. Thus, polarizing ¹²⁹Xe in large quantities generally takes a large amount of laser power. When using a diode laser array, the natural Rb absorption line bandwidth is typically many times narrower than the laser emission bandwidth. The Rb absorption range can be increased by using a buffer gas. Of course, the selection of a buffer gas can also undesirably impact the Rb-noble gas spin-exchange by potentially introducing an angular momentum loss of the alkali metal to the buffer gas rather than to the noble gas as desired.

In any event, after the spin-exchange has been completed, the hyperpolarized gas is separated from the alkali metal prior to introduction into a patient. Unfortunately, after and during collection, the hyperpolarized gas can deteriorate or decay relatively quickly (lose its hyperpolarized state) and therefore must be handled, collected, transported, and stored carefully. Thus, handling of the hyperpolarized gases is critical, because of the sensitivity of the hyperpolarized state to environmental and handling factors and the potential for undesirable decay of the gas from its hyperpolarized state.

Some accumulation systems employ cryogenic accumulators to separate the buffer gas from the polarized gas and to freeze the collected polarized gas. Unfortunately, reductions in polarization of the gas can be problematic as, after final thawing of the frozen gas, the polarization level of the gas can potentially be undesirably reduced by as much as an order of magnitude. Further and disadvantageously, the extremely low operating temperatures of the accumulator near the cryogen source can sometimes clog the collection area of the accumulator, thereby decreasing the rate of, or even preventing, further collection.

### Objects and Summary of the Invention

In view of the foregoing, it is therefore an object of the present invention to provide a cryogenic accumulator and method which can be used in a substantially continuous production environment.

These objects are satisfied by the present invention by a cryogenic accumulator comprising the features of claim 1 and by a method according to claim 22.

In a preferred embodiment, the outer sleeve and the outer wall of the secondary flow channel define a buffer gas exit channel therebetween and the (circumferentially extending) inner wall of the secondary flow channel defines the primary flow channel. It is also preferred that the primary flow channel second end be configured as a nozzle and that the secondary flow channel be configured as a warming or heating jacket to direct circulating room temperature dry gases such as nitrogen therethrough. The circulating nitrogen is separate from the flow channel and acts to compensate or protect the nozzle area against the cold buffer gas exiting along the outside of the primary flow channel and the cryogenic temperatures associated with the cryogen bath. Advantageously, such a secondary flow channel can reduce the likelihood that the primary flow nozzle will freeze and clog from sublimation of the noble gas.

Further and preferably, the accumulator includes first and second isolation valves in communication with the primary flow channel and the buffer gas exit channel. The first isolation valve is positioned at the first end of the primary flow channel and can be used to control the flow of a target gas therethrough. The second isolation valve is positionned spaced-apart from the outer sleeve closed end along the buffer gas exit channel to releasably seal and control the release of buffer gas therethrough. In this embodiment, the accumulator is configured to contain MRI-sized quantities (such as 0.5-2 liters of polarized gas) and is detachably releasable from a hyperpolarizer unit for easy transport to a remote site.

The heating jacket includes an outer wall having opposing first and second ends and an inner wall having opposing first and second ends. The inner wall is spaced apart from the outer wall. The inner wall is configured to be in close proximity to a polarized gas collection path. The jacket also includes a top and bottom which bridge and seal each of the outer and inner walls. The top, bottom and outer and inner walls define at least one enclosed fluid (such as a gas or liquid) circulation channel therebetween. The jacket also includes a fluid and a fluid vent, each of which is in communication with the circulation channel. The fluid inlet and vent are configured to allow flow of a fluid, gas, or gas mixture in the circulation channel.

In a preferred embodiment, the heating jacket fluid inlet is operably associated with a valve such that it is configured to provide a predetermined flow rate of the gas in the circulation channel. It is also preferred that the inner wall circumferentially extends around a center opening to define a flow channel therethrough for a polarized gas.

It is additionally preferred that the inner wall include a first portion which defines a flow channel first diameter and a stepped down portion which defines a flow channel second diameter. In this embodiment, the second diameter is smaller that the first diameter and defines a flow channel nozzle.

### Brief Description of the Drawings

**Figure 1** is a schematic illustration of a hyperpolarizer apparatus.
**Figure 2** is a side perspective view of an accumulator or "cold finger" of the apparatus of **Figure 1** partially immersed in a liquid cryogen according to one embodiment of the present invention.
**Figure 3** is a cross-sectional side view of an accumulator of **Figure 2** according to one embodiment of the present invention.
**Figure 4** is a front view of the accumulator illustrated in **Figure 3**.
**Figure 5** is a cross-sectional side view of an additional embodiment of an accumulator of the present invention.
**Figure 6** is a partial cutaway perspective view of the accumulator illustrated in **Figure 3**.
**Figure 7** is a partial cutaway perspective view of the accumulator illustrated in **Figure 5**.
**Figure 8** illustrates the accumulator of **Figure 7** with heat applied during a thawing process according to one embodiment of the present invention.
**Figure 9** is a block diagram illustrating the steps of a method for accumulating polarized gas.
**Figure 10** is a block diagram illustrating the steps of a method for thawing frozen polarized gas.
**Figure 11** is a block diagram illustrating the steps of a method for extending the useful life of a polarized gas.
**Figure 12A** graphically illustrates polarization levels after thaw versus accumulation flow rates of a polarized gas thawed using a conventional thaw method.
**Figure 12B** graphically illustrates exemplary polarization levels after thaw versus accumulation flow rates of a polarized gas thawed.
**Figure 13** graphically illustrates exemplary polarization levels of polarized gas before freezing and after thawing.
**Figure 13A** graphically illustrates predicted and experimental exemplary polarization levels of polarized xenon corresponding to the polarization flow rate for post-thaw experimental data taken when the xenon is processed.

### Detailed Description of the Preferred Embodiments

The present invention will now be described more fully hereinafter with reference to the accompanying figures, in which preferred embodiments of the invention are shown. This invention may, however, be embodied in many different forms and should not be construed as limited to the embodiments set forth herein. Like numbers refer to like elements throughout. Layers and regions may be exaggerated for clarity. In the description of the present invention that follows, certain terms are employed to refer to the positional relationship of certain structures relative to other structures. As used herein the term "forward" and derivatives thereof refer to the general direction the gas mixture travels as it moves through the hyperpolarizer unit; this term is meant to be synonymous with the term "downstream" which is often used in manufacturing environments to indicate that certain material being acted upon is farther along in the manufacturing process than other material. Conversely, the terms "rearward" and "upstream" and derivatives thereof refer to the directions opposite, respectively, the forward and downstream directions. Also, as described herein, polarized gases are collected, frozen, thawed, and used in MRI spectroscopy or MRI applications. For ease of description, the term "frozen polarized gas" means that the polarized gas has been frozen into a solid state. The term "liquid polarized gas" means that the polarized gas has been or is being liquefied into a liquid state. Thus, although each term includes the word "gas", this word is used to name and descriptively track the gas which is produced via a hyperpolarizer to obtain a polarized "gas" product. Thus, as used herein, the term gas has been used in certain places to descriptively indicate a hyperpolarized noble gas product and may be used with modifiers such as solid, frozen, and liquid to describe the state or phase of that product.

Various techniques have been employed to accumulate and capture polarized gases. For example, U.S. Patent No. 5,642,625 to Cates et al., describes a high volume hyperpolarizer for spin polarized noble gas and U.S. Patent Application No. 08/622,865 to Cates et al. describes a cryogenic accumulator for spin-polarized ¹²⁹Xe. As used herein, the terms "hyperpolarize" "polarize", and the like, mean to artificially enhance the polarization of certain noble gas nuclei over the natural or equilibrium levels. Such an increase is desirable because it allows stronger imaging signals corresponding to better MRI images of the substance and a targeted area of the body. As is known by those of skill in the art, hyperpolarization can be induced by spin-exchange with an optically pumped alkali-metal vapor or alternatively by metastability exchange. *See* Albert et al., U.S. Patent No. 5,545,396.

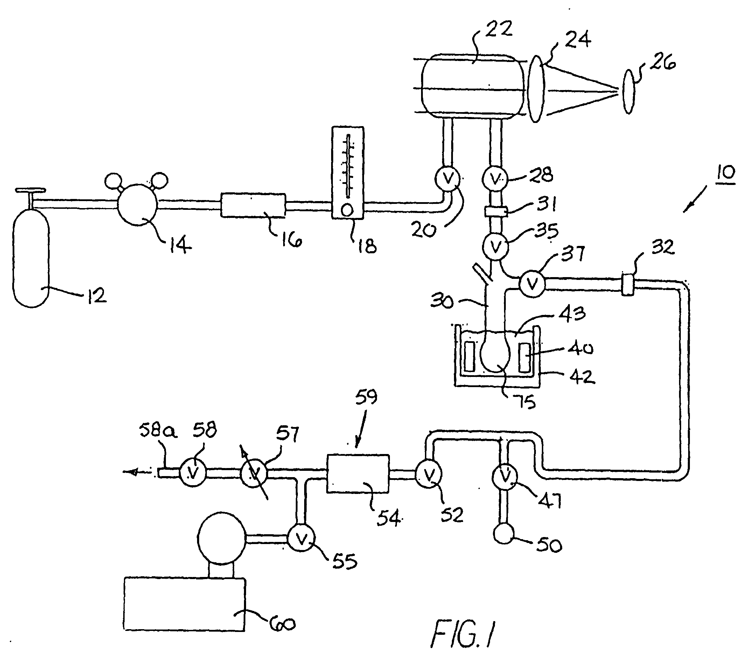

Referring to the drawings, **Figure 1** illustrates a hyperpolarizer unit **10**. This unit is a high volume unit which is configured to continually produce and accumulate spin-polarized noble gases, *i.e.,* the flow of gas through the unit is substantially continuous. As shown, the unit **10** includes a noble gas supply **12** and a supply regulator **14**. A purifier **16** is positioned in the line to remove impurities such as water vapor from the system as will be discussed further below. The hyperpolarizer unit **10** also includes a flow meter **18** and an inlet valve **20** positioned upstream of the polarizer cell **22**. An optic light source such as a laser **26** (preferably a diode laser array) is directed into the polarizer cell **22** through various focusing and light distributing means **24**, such as lenses, mirrors, and the like. The light source is circularly polarized to optically pump the alkali metals in the cell **22**. An additional valve **28** is positioned downstream of the polarizer cell **22**.

Next in line, as shown in **Figure 1**, is a cold finger or accumulator **30**. The accumulator **30** is connected to the hyperpolarizer unit **10** by a pair of releasable mechanisms such as threaded members or quick disconnects **31**, **32**. This allows the accumulator to be easily detached, removed, or added, to and from the system **10**. The accumulator **30** is operably associated with a cold source or refrigeration means **42**. Preferably, and as shown, the cold source **42** is a liquid cryogen bath **43**. The accumulator will be discussed in more detail hereinbelow.

A vacuum pump **60** is in communication with the system. Additional valves to control flow and direct exit gas are shown at various points (shown as **52**, **55**). A shut-off valve **47** is positioned adjacent an "on-board" exit gas tap **50**. Certain of the valves downstream of the accumulator **30** are used for "on-board" thawing and delivery of the collected polarized gas as will be described further below. The system also includes a digital pressure transducer **54** and a flow control means **57** along with a shut-off valve **58**. The shut-off valve **58** preferably controls the flow of gas through the entire system or unit **10**; it is used to turn the gas flow on and off, as will be described below. As will be understood by those of skill in the art, other flow control mechanisms, devices (analog and electronic) may be used.

In operation, a gas mixture is introduced into the system at the gas source **12**. As shown in **Figure 1**, the source **12** is a pressurized gas tank which holds a pre-mixed gas mixture. The gas mixture includes a lean noble and buffer gas mixture gas (the gas to be hyperpolarized is present as a relatively small amount in the premixed gas mixture). Preferably, for producing hyperpolarized ¹²⁹Xe, the pre-mixed gas mixture is about 95-98% He, about 5% or less ¹²⁹Xe, and about 1% N₂.

It is also preferred that the pre-mixed gas mixture comprises a minimal amount of the xenon -131 (or ¹³¹ Xenon) isotope (reduced from its natural levels). In nature, the typical xenon isotopic abundances are as follows:

**Table I**

| **Isotope** | **Abundance** | **Nuclear Spin** |
|---|---|---|
| ¹²⁴Xe | 0.1% | 0 |
| ¹²⁶Xe | 0.09% | 0 |
| ¹²⁸Xe | 1.91% | 0 |
| ¹²⁹Xe | 26.4% | ½ |
| ¹³⁰Xe | 4.1% | 0 |
| ¹³¹Xe | 21.2% | 3/2 |
| ¹³²Xe | 26.9% | 0 |
| ¹³⁴Xe | 10.4% | 0 |
| ¹³⁶Xe | 8.9% | 0 |

"Enriched" ¹²⁹Xe mixtures are used to provide sufficient amounts of the ¹²⁹Xe gas for the hyperpolarized gas mixture. As used herein, the form "enriched" means increasing the abundance of ¹²⁹Xe over its natural abundance level. However, the enriched ¹²⁹Xe typically also includes other Xenon isotopes. Unfortunately, at least one particular isotope - ¹³¹Xe can interact with frozen ¹²⁹Xe (particularly at low temperatures such as 4.2° K) in a manner which can cause the ¹²⁹Xe to depolarize. At low temperatures, the ¹³¹Xe acts like a "spin-sink" to absorb or decay the ¹²⁹Xe polarization and becoming a potentially dominant relaxation mechanism at the crystal grain boundaries of the frozen "solid" ¹²⁹Xe polarized gas.

As shown in Table I above, ¹³¹Xe is an isotope with a nuclear spin greater than one-half. As such, it has a "quadruple moment" which means that ¹³¹Xe is able to relax by interacting with electric field gradients. *See* Gatzke et al., *Extraordinarily slow nuclear spin relation in frozen laser-polarized* ^{*129*}*Xe*, Phys. Rev. Lett. 70, pp. 690-693 (1993).

It has been suggested that at 4.2° K, the dominant solid phase relaxation mechanism is "cross-relaxation" between the ¹²⁹Xe and ¹³¹Xe isotopes at the crystal grain boundaries. In addition, where the "frozen" or "solid" ¹²⁹Xe gas takes on a form similar to a flake (such as a snowflake) the form has a relatively large surface area. Unfortunately, this relatively large surface area can also allow greater depolarizing interactions with the ¹³¹Xe. It is believed that the greatest or "most-efficient" interchange is at the crystal grain boundaries because that is typically where the electric fields are strongest. This electric field strength can then allow the ¹³¹Xe nuclear spin flip energy to become nearly the same as the ¹²⁹Xe nuclear spin flip energy.

Examples of enriched ¹²⁹Xe mixtures with a reduced ¹³¹Xe isotope content are given below.

### Example 1 82.3% Enriched ¹²⁹Xe Gas Mixture.

| **Isotope** | **Abundance** | **Nuclear Spin** |
|---|---|---|
| ¹²⁴Xe | 0.47% | 0 |
| ¹²⁶Xe | 0.43% | 0 |
| ¹²⁸Xe | 8.41% | 0 |
| ¹²⁹Xe | 82.3% | ½ |
| ¹³⁰Xe | 4.52% | 0 |
| ¹³¹Xe | 3.45% | 3/2 |
| ¹³²Xe | 0.36% | 0 |
| ¹³⁴Xe | 0.01% | 0 |
| ¹³⁶Xe | 0.01% | 0 |

### Example 2 47.2% Enriched ¹²⁹Xe Gas Mixture.

| **Isotope** | **Abundance** | **Nuclear Spin** |
|---|---|---|
| ¹²⁴Xe | 0.14% | 0 |
| ¹²⁶Xe | 0.28% | 0 |
| ¹²⁸Xe | 52.0% | 0 |
| ¹²⁹Xe | 47.2% | ½ |
| ¹³⁰Xe | 0.22% | 0 |
| ¹³¹Xe | 0.09% | 3/2 |
| ¹³²Xe | 0.03% | 0 |
| ¹³⁴Xe | 0.02% | 0 |
| ¹³⁶Xe | 0.02% | 0 |

In a preferred embodiment, when the collected polarized ¹²⁹Xe will be exposed to cold temperatures and frozen, the enriched ¹²⁹Xe gas mixture preferably includes less than about 3.5% ¹³¹Xe, and more preferably less than about 0.1% ¹³¹Xe.

In any event, the gas "enriched" mixture is passed through the purifier 16 and introduced into the polarizer cell **22.** The valves **20, 28** are on/off valves operably associated with the polarizer cell **22**. The gas regulator **14** preferably steps down the pressure from the gas tank source **12** (typically operating at 13,780.2kPa (2000 psi or 136 atm)) to about 608 - 1013.25 kPa (6-10 atm) for the system. Thus, during accumulation, the entire manifold (conduit, polarized cell, accumulator, etc.) is pressurized to the cell pressure (about 608 - 1013.25 kPa (6-10 atm)). The flow in the unit **10** is activated by opening valve **58** and is controlled by adjusting the flow control means **57**.

The typical residence time of the gas in the cell **22** is about 10-30 seconds; *i.e.,* it takes on the order of 10-30 seconds for the gas mixture to be hyperpolarized while moving through the cell **22**. The gas mixture is preferably introduced into the cell **22** at a pressure of about 608 - 1013.25 kPa (6-10 atm). Of course, with hardware capable of operating at increased pressures, operating pressures of above 1013.25 kPa (10 atm), such as about 2026.5- 3039.75 kPa (20-30 atm) are preferred to pressure broaden the Rb and absorb up to 100% of the optical light. In contrast, for laser linewidths less than conventional linewidths, lower pressures can be employed. The polarizer cell **22** is a high pressure optical pumping cell housed in a heated chamber with apertures configured to allow entry of the laser emitted light. Preferably, the hyperpolarizer unit **10** hyperpolarizes a selected noble gas such as ¹²⁹Xe (or ³He) via a conventional spin-exchange process. A vaporized alkali metal such as rubidium ("Rb") is introduced into the polarizer cell **22**. The Rb vapor is optically pumped via an optic light source **26**, preferably a diode laser.

The unit **10** employs helium buffer gas to pressure broaden the Rb vapor absorption bandwidth. The selection of a buffer gas is important because the buffer gas -- while broadening the absorption bandwidth -- can also undesirably impact the alkali metal-noble gas spin-exchange by potentially introducing an angular momentum loss of the alkali metal to the buffer gas rather than to the noble gas as desired. In a preferred embodiment, ¹²⁹Xe is hyperpolarized through spin exchange with the optically pumped Rb vapor. It is also preferred that the unit **10** uses a helium buffer gas with a pressure many times greater than the ¹²⁹Xe pressure for pressure broadening in a manner which minimizes Rb spin destruction.

As will be appreciated by those of skill in the art, Rb is reactive with H₂O. Therefore any water or water vapor introduced into the polarizer cell **22** can cause the Rb to lose laser absorption and decrease the amount or efficiency of the spin-exchange in the polarizer cell **22**. Thus, as an additional precaution, an extra filter or purifier (not shown) can be positioned before the inlet of the polarizer cell **22** with extra surface area to remove even additional amounts of this undesirable impurity in order to further increase the efficiency of the polarizer.

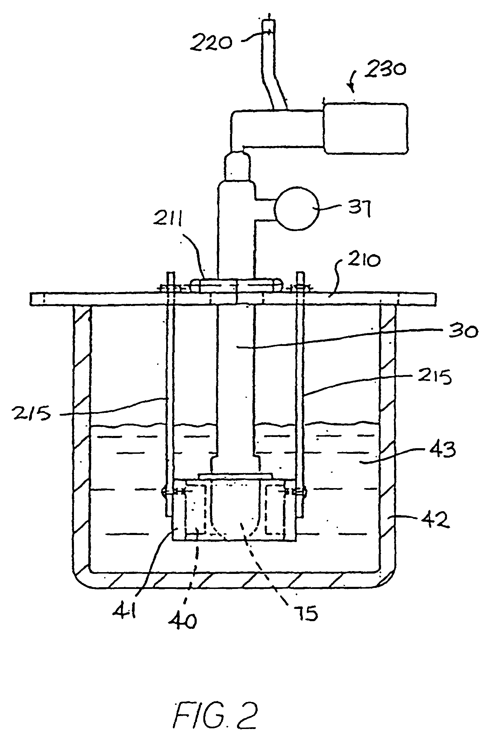

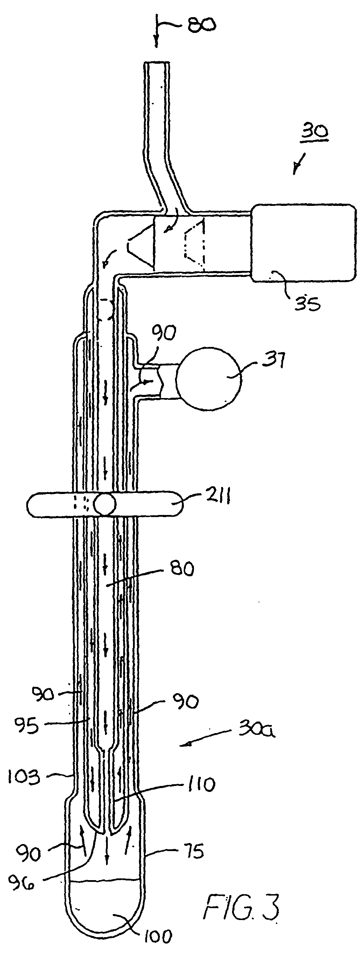

Hyperpolarized gas, together with the buffer gas mixture, exits the polarizer cell **22** and enters the accumulator **30**. Referring now to **Figures 3-7**, the polarized gas and buffer gas are directed down a primary flow path **80** and into a collection reservoir **75** located at the bottom of the accumulator **30**. In operation, at the lower portion of the accumulator **30a**, the hyperpolarized gas is exposed to temperatures below its freezing point and collected as a frozen product **100** in the reservoir **75**. The remainder of the gas mixture remains gaseous and exits the primary flow path **80** and the reservoir **75** by counterflowing in an exit path **90** different from the primary flow path **75** such that it is directed out of the accumulator **30**. The accumulator **30** will be discussed in more detail below. The hyperpolarized gas is collected (as well as stored, transported, and preferably thawed) in the presence of a magnetic field, generally on the order of at least 500 Gauss, and typically about 2kiloGauss, although higher fields can be used. Lower fields can potentially undesirably increase the relaxation rate or decrease the relaxation time of the polarized gas. As shown in **Figure 2**, the magnetic field is provided by permanent magnets **40** positioned about a magnetic yoke **41**.

The hyperpolarizer unit **10** can also use the temperature change in the outlet line between the heated pumping cell **22** and the refrigerated cold trap or accumulator **30** to precipitate the alkali metal from the polarized gas stream in the conduit above the accumulator **30**. As will be appreciated by one of skill in the art, the alkali metal can precipitate out of the gas stream at temperatures of about 40 °C. The unit can also include an alkali metal reflux condenser (not shown). Preferably, the refluxing condenser employs a vertical refluxing outlet pipe, which is kept at room temperature. The gas flow velocity through the refluxing pipe and the size of the refluxing outlet pipe is such that the alkali metal vapor condenses and drips back into the pumping cell by gravitational force. In any event, it is desirable to remove the alkali metal such that the product is non-toxic and comply with regulatory standards (for example at least to a level at or below 10 ppb) prior to delivering polarized gas to a patient.

Optionally, an intermediate cold trap can also be positioned between the exit of the polarizer cell **22** and the cold finger **30**. The temperature of the intermediate cold trap (not shown) will preferably be designed to trap out any alkali metal (e.g. Rb) while leaving the noble gas and carrier gas (es) free to reach the cold finger **30**. This can be important for in vivo applications where it is important to remove the Rb from the hyperpolarized gas (*i.e*., remove the Rb to a level such that no more than trace amounts such as on the order of one ppb or less remains in the hyperpolarized gas when delivered to a patient).

Once a desired amount of hyperpolarized gas has been collected in the accumulator **30**, the accumulator can be detached or isolated from the system. In a preferred embodiment, valve **28** is closed, leaving the cell **22** pressurized. This allows the accumulator **30** and the downstream plumbing to begin to depressurize because the flow valve **58** is open. Preferably, the unit **10** downstream of the valve **28** is allowed to depressurize to about 1.5 atm before the flow valve **58** is closed. After closing the flow valve **58**, valve **55** can be opened to evacuate the remaining gas in the manifold. Once the outlet plumbing is evacuated, valves **35** and **37** are closed. If the collected gas is to be distributed "on board", *i.e.,* without removing the accumulator **30** from the unit **10,** a receptacle such as a bag or other vessel can be attached to the outlet **50**. The valve **47** can be opened to evacuate the attached bag (not shown). Once the bag is evacuated and the gas is ready to thaw, valve **52** can be optionally closed. This minimizes the contact of the polarized gas with the pressure transducer region **59** of the unit **10**. This region typically includes materials that have a depolarizing effect on the polarized gas. Thus, long contact times with this region may promote relaxation of the polarized gas.

If the valve **52** is not closed, then valve **55** is preferably closed to prevent the evacuation of polarized thawed gases. It is also preferred that the flow channels on the downstream side of the cell **22** are formed from materials which minimize the decaying effect on the polarized state of the gas. Coatings can also be used such as those described in U.S. Patent No. 5,612,103, the disclosure of which is hereby incorporated by reference as if recited in full herein. In the "on-board" thaw operation, valve **37** is opened to let the gas out. It then proceeds through valve **47** and exits outlet **50**.

In the "detached" or "transported accumulator" thaw mode, accumulator first and second isolation valves **35**, **37** are closed after the depressurization and evacuation of the accumulator **30**. Evacuating the accumulator **30** allows any residual gas in the accumulator to be removed. Leaving residual gas in the accumulator **30** with the frozen polarized gas may contribute to the heat load on the frozen gas, possibly raising the temperature of the frozen gas and potentially shortening the relaxation time. Thus, in a preferred embodiment, after depressurization and evacuation and closing the isolation valves **35**, **37**, the accumulator **30** is disconnected from the unit **10** via release points **31**, **32**.

It is also preferred that the accumulator include O-rings in grooves (**Figure 2, 220**) to assist in sealing the quick connects (or other attaching means) to the conduit lines in the system. This type of O-ring/groove sealing mechanism can help assure the seals integrity even at the elevated operating pressures (*i.e*., 608 - 1013.25 kPa (6-10 atm) and greater) of the unit.

The isolation valves **35, 37** are in communication with the primary flow channel **80** and the buffer gas exi channel **90** respectively and each can adjust the amount of flow therethrough as well as close the respective paths to isolate the accumulator from the system **10** and the environment. After the filled accumulator **30** is removed, another accumulator can be easily and relatively quickly attached to the release points **31**, **32**. Preferably, when attaching the new accumulator **30**, the outlet manifold is evacuated using valve **55** (with valves **52**, **35**, **37** open). When a suitable vacuum is achieved (such as about 13.3Pa (100milliTorr)) which typically occurs within about one minute or so, valve **55** is closed. Valve **28** is then re-opened which repressurizes the outlet manifold to the operating cell pressure. Valve **58** is then opened to resume flow in the unit **10**. Preferably, once flow resumes, liquid nitrogen is applied to the accu mulator **30** to continue collection of the hyperpolarized gas. Typically such a changeover takes on the order of less than about five minutes. Thus, a preferred hyperpolarizer unit **10** is configured to provide a continuous flow of hyperpolarized ¹²⁹Xe gas for continuous production and accumulation of same.

Turning now to **Figure 2,** an accumulator and magnet yoke assembly **230** is shown. The accumulator **30** is supported by a support platform **210** positioned over the cryogen bath **43**. A pair of plates **215** longitudinally extent from the support platform **210** and connect to the magnet yoke **41**. The magnet yoke **41** is positioned adjacent to and in close proximity to the collection reservoir **75** of the accumulator **30** to provide the desired magnetic field to the collected polarized gas. As shown, the accumulator **30** includes a support contact portion **211**, which is configured to rest against the support platform **210**.

### The Accumulator

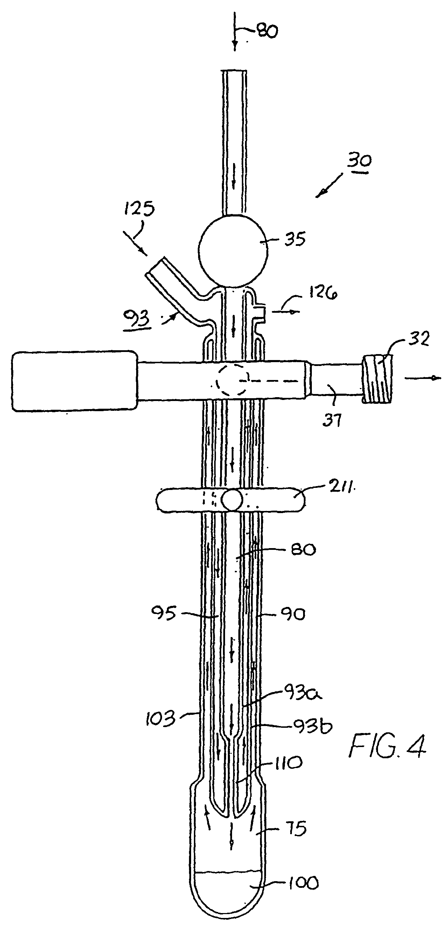

**Figures 3** and **4** show one embodiment of an accumulator **30** according to the instant invention. As shown the accumulator **30** includes a central primary flow path **80**, a secondary flow path **95**, and an exit buffer gas channel **90**. The secondary flow path or channel **95** is positioned intermediate of the primary flow path channel **80** and the buffer exit channel **90**. In a preferred embodiment, the accumulator **30** includes a nozzle **110** at the lower end of the primary flow path. The nozzle **110** can help improve localization of the hyperpolarized gas as it impacts the cold surfaces of the reservoir **75**. The nozzle **110** may also allow Joule-Thompson expansion of the cooling of the gas stream to well below the freezing point of the hyperpolarized gas, advantageously minimizing the heat load on the stationary and collected hyperpolarized gas and thereby, potentially lengthening its relaxation time. In any event, the accumulator **30** is preferably immersed in the cryogen bath **43** such that the reservoir **75** and about 7.62-15.24 cm (3-6 inches) of the tube is immersed. If submerged in liquid nitrogen, the exterior wall of the outer sleeve **103** and the exterior wall or the reservoir **75** will be at about 77°K. The freezing point of Xenon is approximately 160°K. Thus, upon exiting the primary flow path **80**, the hyperpolarized gas hits the cold surface and freezes into the reservoir **75** while the buffer gases exit the accumulator via the exit channel **90**. The reservoir can include a surface coating to help prevent relaxation caused by the polarized gas's contact with same. *See U.S.* Patent No. 5,612,103, *"Improved Coatings for the Production of Hyperpolarized Noble Gases"*. Alternatively, the container can be formed from or include other materials such as high purity non-magnetic metallic films.

As shown in **Figure 4**, the secondary flow path **95** has an inlet and outlet **125**, **126**, respectively, positioned about 180° apart at a top portion of the accumulator **30**. Of course, as will be appreciated by one of skill in the art, alternative arrangements of the secondary flow path inlet and outlet **125, 126** can also be employed. Preferably, the inlet and outlet **125**, **126** are configured to be above the cryogen bath **43** or other refrigeration means when the accumulator **30** is assembled thereto. Except for its respective inlet and vent ports **125**, **126**, the secondary flow path **95** is enclosed and separate from the primary flow path **80** and the exit gas path **90**. As such, the secondary flow path **95** includes a sealed closed end **96**.

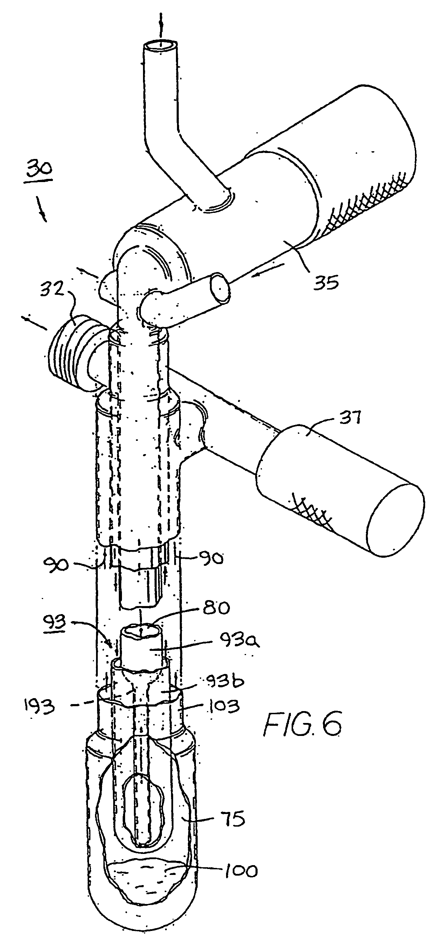

In operation, as shown in **Figure 6**, the secondary flow path **95** provides heat to a region of the accumulator **30**. Preferably, the secondary flow path defines a heating jacket **93**. The heating jacket **93** is configured to provide a contained warm stream of a fluid, preferably a gas, around the primary flow path **80**. More preferably, the heating jacket **93** directs warm or ambient temperature nitrogen down the secondary flow path to an area adjacent the lower portion of the primary path **80**; that is, the portion of the secondary path is in close proximity to or adjacent the reservoir **75**. In a preferred embodiment, the warming gas in the heating jacket **93** is directed to the nozzle **110** area of the primary flow path **80** via the secondary flow path **95**. Advantageously, such a warming gas can compensate for the undesirable tendency of this area of the primary flow path to freeze and clog due to frozen gases trapped in the flow path **80**. Further and advantageously, this configuration can also minimize any associated heat load which is directed into the reservoir **75** and on the collected frozen polarized gas. The clogging problem can be particularly troublesome in accumulators with nozzle designs, as even small amounts of build up in the reduced exit area of the nozzle **110** can block the primary flow path **80** and decrease and even prevent further collection of polarized gas. "Warming" as used herein can be the application of heat at any temperature above the freezing point of selected polarized gas, *i.e.* above 160°K for ¹²⁹Xe.

Generally stated, the relaxation time of solid polarized gas (especially ¹²⁹Xe) is strongly dependent on the temperature of the frozen gas. Stated differently, the lower the temperature of the frozen gas, the longer the relaxation time. Thus, it is important to minimize the heat load on the accumulated frozen gas. The heat load presented by the gas stream directed down the primary flow path **80** is largely attributed to the need to cool the buffer gas from room temperature to the cryogenic temperature (as described herein liquid nitrogen (LN₂) or 77°K. This heat load is estimated to be on the order of 2W. Thus, in order to minimize the heat load on the accumulated polarized ¹²⁹Xe, it is desirable to cool the gas steam to close to (but above) the freezing temperature of the polarized gas prior to the exit point of the nozzle **110**. For ¹²⁹Xe, the buffer gas is preferably cooled to just above 160°K, below which the Xe can freeze in the nozzle potentially causing a clog or blockage. Advantageously, cooling the exit gas to 160°K can cut the heat load on the frozen polarized gas by as much as 50%. The configuration of the instant invention allows this exit channel to be so cooled through the counter-flow of the buffer gas. Advantageously, this cooling counter-flow does not overly expose the nozzle **110** to low temperatures because the nozzle **110** or most susceptible area of the flow path **80** is separated from the exit channel by the heating jacket or secondary flow channel **95**.

Referring again to **Figure 4**, as shown, the primary flow path **80** is defined by the shape of the inner wall **93a** of the heating jacket **93**. Preferably, the inner wall **93a** circumferentially extends around an opening to define the primary flow path **80**. Similarly, the outer wall **93b** of the heating jacket **93** together with the outer sleeve **103** of the accumulator **30** defines the buffer exit path **90**. As shown in **Figure 6**, in a preferred embodiment, the inner wall **93a**, the outer wall **93b** and the outer sleeve **103** are radially aligned. The inner wall of the heating jacket **93** includes a stepped down portion **193** with a diameter less than the diameter of the preceding section of the inner wall. This stepped down portion is configured to provide the nozzle **110** in the primary flow path **80.**

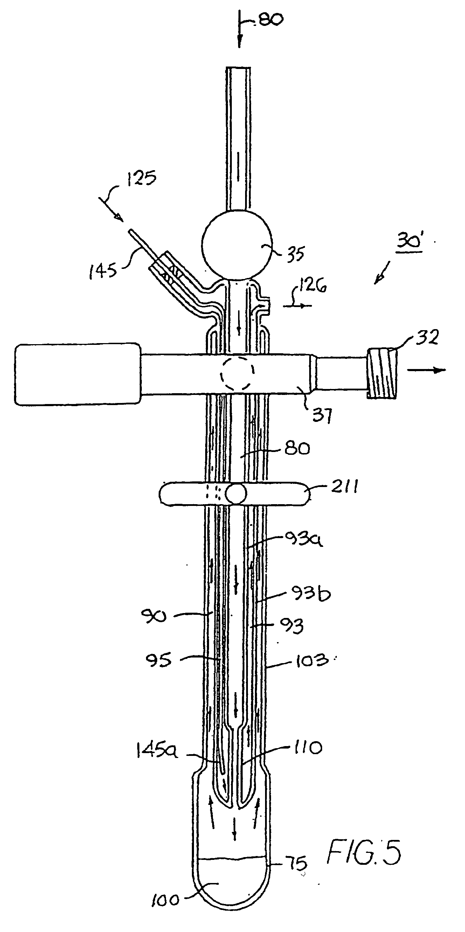

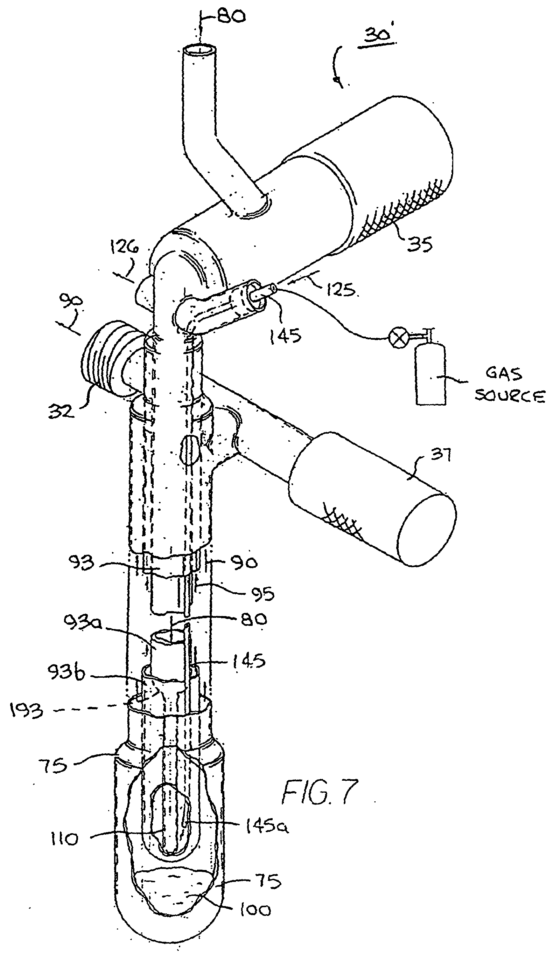

**Figures 5** and **7** illustrate a preferred embodiment of an accumulator **30'** according to the instant invention. As shown in this embodiment, the heating jacket **93** includes at least one elongated conduit **145** which extends along a major portion of the secondary flow path **95**. As the conduit **145** is exposed to cryogenic temperatures, it should be made from suitable substantially non-depolarizing and cryo-accepting materials such as PTFE and the like. Suitable materials include materials which have a low temperature resistance. One example of a brand of such a material is TEFLON™ or metallic-film coated surfaces. The conduit **145** directs the warming gas down to the lower portion of the primary flow path **80**, and more preferably directs the warming gas to the nozzle area **110** of the primary flow channel above the reservoir **75**. As such, the lower end **145a** of the conduit is preferably positioned adjacent the nozzle **110**. Once released, the warming gas travels up the circumferentially extending secondary flow path **95** and exits at the outlet vent **126**. This warming gas can counteract the cold/clogging effect the counter-flow of the cold buffer gas has on the primary flow path in the region susceptible to clogging as discussed above. Of course, additional heating jacket inlets, conduits, and vents (not shown) can also be employed within the scope of the invention.

Examples of suitable diameters of the primary flow path **80**, the secondary flow path **95**, and the buffer gas exit channel **90** are 6.35, 12.7, and 19.05mm (0.25, 0.50, and 0.75 inches), respectively. In one embodiment the nozzle **110** extends along the primary flow path for about 25.4mm (1.0 inches). Preferably, the accumulator **30** is formed from glass such as PYREX™ and is configured to withstand from about 608 - 1013.25 kPa (6-10 atm) or more of pressure.

In operation, it is preferred that, during accumulation of frozen hyperpolarized gas, the warming gas is introduced into the secondary channel at a rate of about 7.8658 -47.2 ml/s (1-6 ft³/hour), more preferably at the rate of about 15.732-39.329 ml/s (2-5 ft³/hr), and still more preferably at a rate of about 23.597 ml/s (3 ft³/hr). Preferably, during collection, the accumulator **30** operates at the same pressure as the optical pumping cell.

As discussed above, the preferred warming gas is a dry ambient temperature N₂ (N₂ has approximately two times the heat capacity of helium), but the invention is not limited thereto. Exemplary preferred temperatures of the warming gas are from about 10-26.7° C (50°-80°F), and more preferably from about 20-25.6° C (68°-78°F). In a preferred embodiment, a corresponding "heating gas" flow rate is set to a minimum level corresponding to a predetermined temperature of the warming gas; *i.e.,* the minimum rate is set for a certain temperature below which a clog occurs, this minimum rate can be termed the "critical flow rate". If higher temperatures are used, lower flow rates will typically be required. Examples of other warming gases include, but are not limited to, helium, dry air, and the like. Preferably, if higher temperature "warming" gases are used a lower corresponding flow rate is used. In contrast, if lower temperature "warming" gases are used then a higher corresponding flow rate is used.

Advantageously, the instant invention can collect about 80-100% of the polarized gas in the gas stream. In addition, the instant invention can yield a polarized gas product with an extended useful life. This is attributed to the improved collection and/or thawing techniques which can yield a polarized gas product which retains greater polarization levels compared to conventional techniques as will be discussed further below.

### Thawing

As noted above, a preferred embodiment of the instant invention employs a compact permanent magnet arrangement positioned around the hyperpolarized gas. Unfortunately, the magnetic field provided by such an arrangement can be somewhat inhomogeneous. As gas is thawed, this inhomogeneity can depolarize the hyperpolarized gas relatively quickly. Freshly thawed ¹²⁹Xe is particularly susceptible to inhomogeneity induced decay ("loss of polarization"). For example, relaxation of gaseous ¹²⁹Xe is particularly troublesome as it diffuses through inhomogeneous fields. This relaxation generally scales linearly with inverse pressure of the gas. That is, at low gas pressures, which occur at the beginning of the thawing process, the inhomogeneity (field gradients) induced relaxation effect is the strongest. (Relaxation of ¹²⁹Xe at 101, 325 kPa (1 atm) of gas pressure has been measured at just 22 seconds). This problem is solved by closing the isolating valves **35**, **37** in the accumulator **30** during the initial thaw. As the polarized gas thaws, pressure builds up rapidly, quickly exceeding 1 atm and building further. As the pressure rises, the remaining solid ¹²⁹Xe goes into liquid form rather than gaseous form. The liquid ¹²⁹Xe is relatively insensitive to magnetic field gradients, inhomogeneity relaxation, temperature effects, and magnetic field strengths, thus making it one of the more robust forms of hyperpolarized ¹²⁹Xe. Liquid ¹²⁹Xe has typical relaxation times of about 20-30 minutes. *See* K.L. Sauer et al., *Laser Polarized Liquid Xenon*, Appl. Phys. lett. (Accepted 1997). The liquid state further helps to quickly distribute heat to the remaining solid ¹²⁹Xe, thus further speeding the thaw.

The heating jacket **93** can also improve the thawing process of the frozen polarized gas. It is important to rapidly transform the frozen polarized gas into a liquid state as both the solid and the gas states of Xenon are extremely sensitive to depolarization during the transition. For example, as solid or frozen ¹²⁹Xe is warmed to near its melting point, the relaxation time is dramatically reduced from 3 hours at 77°K to just a few seconds near the phase transition point. In addition, gaseous relaxation at temperatures just above the sublimation temperature of ¹²⁹Xe is rapid, with an exponential dependence on temperature. For example, the relaxation time of gaseous ¹²⁹Xe on a given surface at 160°K is only 3% as long as that at 300°K on the same surface. Further, during the early stages of thawing when the Xe gas pressure is low, the gaseous ¹²⁹Xe is more susceptible to the inhomogeneity problems discussed above.

Conventionally, heat has been supplied to the exterior of the accumulator during thawing. As the frozen hyperpolarized gas began to thaw it would freeze again, such as on the exit point of the primary flow path **80**. This could cause the ¹²⁹Xe to freeze and thaw more than once during the thawing process, as well as causing the polarized gas product to spend more time around the sensitive transition phase where relaxation is more rapid.

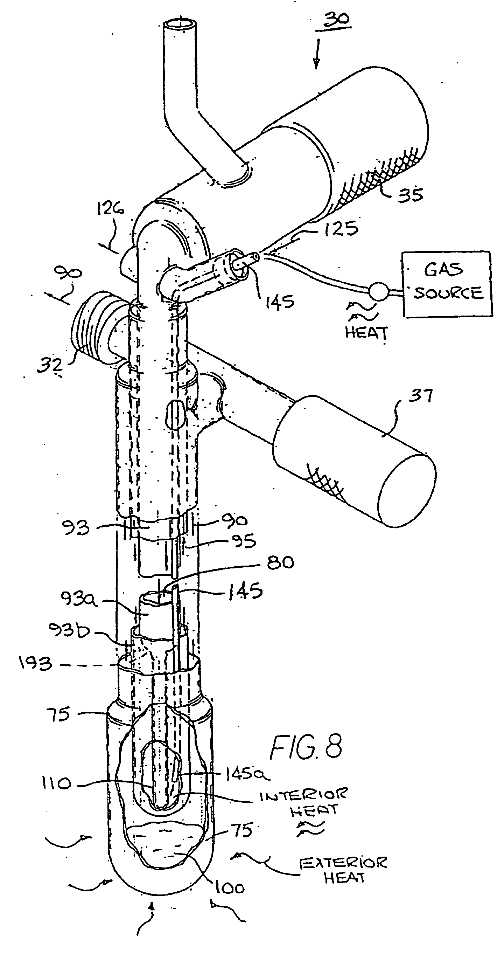

Advantageously, the heating jacket **93** of the accumulator **30**, **30'** described above can additionally improve the thawing process. Turning to **Figure 8**, the heating jacket or secondary flow channel **95** of the accumulator can supply heat to the nozzle area **110** of the accumulator **30** during the thawing process. Preferably the lower area of the flow path or the nozzle area is preheated before thawing so that the nozzle **110** is well above the freezing point of the polarized gas prior to applying heat to the external surface of the reservoir **75**. It is additionally preferred, that during the thawing, heat is supplied to both the exterior and the interior of the cold finger. The interior heating being preferably applied to the lower region of the accumulator, *i.e.,* the nozzle area. The nozzle **110** is thus warmed by the circulating fluid (preferably gas) in the heating jacket **93**. Various warming gases such as those described above can be used. Preferably, the flow rate of the warming gas is higher than that used during the accumulation process, such as about 39.329 - 94.39 ml/s (5-12 ft³/hr), and more preferably at about 78.658 ml/s (10 ft³/hr) during thaw. Similarly, the preferred temperatures of the "warming" gas supplied during thawing are at typical internally controlled ambient conditions (for example room temperature gases such as 20-25.6°C (68-78°F)).

For a "transported" accumulator **30**, once all the ¹²⁹Xe is liquid, the isolation valve **35** is preferably opened leading to an attached evacuated chamber or bag or other delivery means or collection vessel. Of course either of the valves **35**, **37** can be opened depending on where the delivery vessel or receptacle is attached (not shown). For the "on-board" accumulator, isolation valve **37** is the operative valve as described above. The sudden decrease in pressure causes the liquid ¹²⁹Xe to become gaseous and exit the accumulator **30** rapidly, advantageously thereby spending a minimum amount of time in the inhomogeneous magnetic field in the gaseous state. Similarly, if the "on-board" release is employed, the isolation valve **37** is opened and the gas flows through valve **47** and exits outlet **50** into a delivery vessel. Conventional methods of thawing included opening the cold finger (accumulator) to the vessel to be filled and then starting the thaw. This thaw could typically take 30 seconds or more to complete for single patient dose amounts. In comparison and advantageously, the instant thaw method can be completed in less than about 10 seconds, and preferably in less than about 5-6 seconds for single dose amounts of frozen hyperpolarized gas. A typical patient dose is from about 0.20-1.25 liters ("L") and preferably about 0.5-1.0 L. The conversion weight is about 5.4 grams /L of Xe. Similarly, the density of solid Xe is about 3.1g/cm³, and a corresponding patient volume of polarized frozen Xe can be calculated at about 1.8cm³/L.

Advantageously, observations of the instant thawing method indicate a reliable factor of about 2 or more improvement in the final polarization level of thawed ¹²⁹Xe as compared to that thawed by conventional methods.

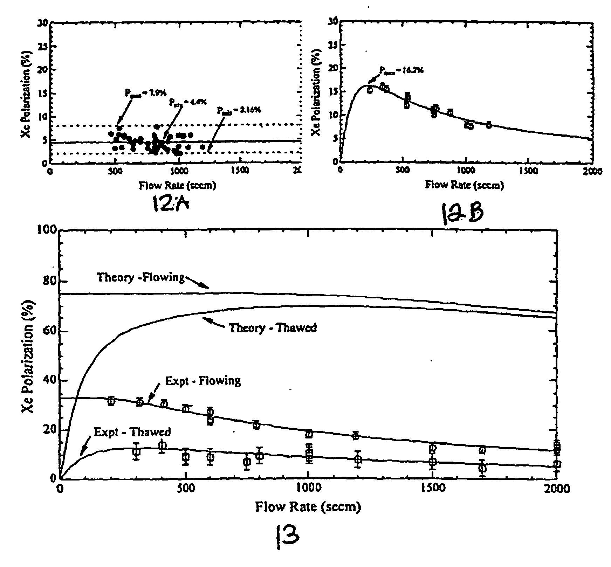

Referring now to **Figures 12A** and **12B, Figure 12A** illustrates the polarization results obtained by a conventional thaw technique while **Figure 12B** graphically illustrates results obtained by the improved thaw method as described above. Each of the graphs plot % polarization of ¹²⁹Xe after thaw in relationship to the total gas flow rate through the polarization cell **22** (and therefore the entire unit). The corresponding ¹²⁹Xe flow rate is the % of the total gas mix. In the example shown, ¹²⁹Xe makes up about 1% of the total gas mix, thus the ¹²⁹Xe-flow rate is the total flow rate divided by 100. For example at a flow rate of 1000 standard cm³ (standard cubic centimeters per minute ("sccm")), ¹²⁹Xe is typically accumulated at the rate of 10 cm³ per min or 600 cm³ per hour. Higher flow rates are desired to increase the through-put of ¹²⁹Xe. However, polarization is reduced at higher flow rates. This is attributed to the reduced time that the ¹²⁹Xe spends in residence time in spin exchange contact with the optically pumped Rb at higher flow rates. That is, the Xe residence time in the cell **22** can generally be described mathematically as equal to the gas pressure multiplied by the cell volume divided by the flow rate (*PV*/*m*).

**Figure 12A** shows the conventional thaw technique yields scattered polarization results which are attributed to random polarization losses mainly occurring during thawing. **Figure 12B** tracks with the optical pumping characteristics described above and now produces predictable post-thaw polarization levels corresponding to the accumulation flow rate.

As shown in **Figure 12B**, when thawing according to the improved method described above (under pressurization and with internal and external heating), for flow rates below 1000 sccm (or standard cm³/min), polarization levels after thaw of above 10% are reliably achieved. The results shown in this figure represent a 190 cm³ volume of ¹²⁹Xe (and Rb polarization levels of about 0.25-0.49). Of course, as will be appreciated by one of skill in the art, different volumes *(i.e.,* larger or smaller) of the polarized gas will have different relative values associated therewith. For example, larger volumes of ¹²⁹Xe take longer times to polarize, therefore at the same flow rates, the polarization of the larger volume will be less than that shown in **Figure 12B**. Stated differently, for larger amounts of polarized gas, the associated polarization curve will drop below the values shown relative to that for an exemplary 190 cm³ volume of polarized gas as shown in **Figure 12B.** Also, typically, larger amounts of polarized gas can result in a larger loss attributed to solid-phase relaxation. However, as shown by the graph, frozen gas thaw method which results in a post-thaw polarization curve which predictably follows the initial polarization curve. In contrast, as shown by **Figure 12A,** the conventional polarization level after thaw is highly unpredictable, with an average of about 4.4%. Indeed, at about 900 sccm (standard cm³/min), the polarization point is about 2.16% while prediction is 18.7%, thus making the retention fraction a low 12.2% (losing about 87.8% of the starting polarization). Unlike the conventional method, the method produces polarization levels after thaw that predictably corresponds to the flow rate used during accumulation.

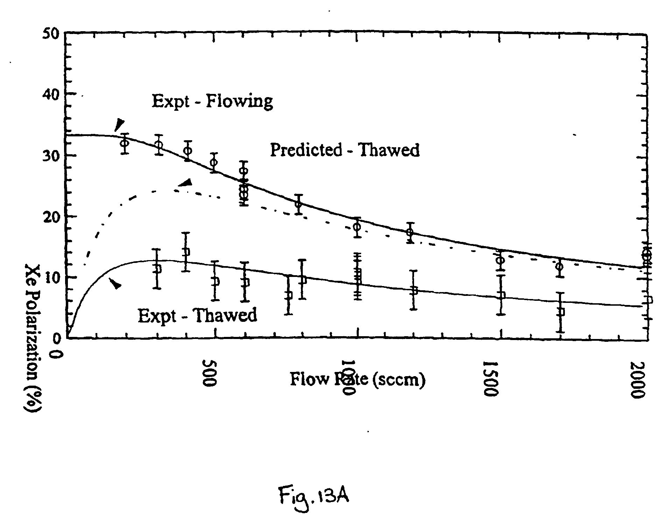

**Figure 13** illustrates experimental and theoretical polarization levels before and after thawing. The experimental flowing curve shows the polarization levels achieved before freezing (the level measured as the ¹²⁹Xe exits the pumping cell **22**). The experimental data points on the graph represent thawed data points achieved by thawing the collected, frozen polarized gas according to the present invention. The experimental data confirms that the methods improve the predictability of the polarization retention fraction now achievable as well as increases the value of the polarization retention fraction (amount of polarization retained post-thaw relative to that attained prior to freeze).

**Figure 13A** illustrates a flow curve used to predict polarization levels as expected from a thawed polarized xenon product, this curve representing post-thaw polarization levels achievable absent polarization losses during freezing and thawing. This curve includes losses from normal relaxation of solid Xe (which can be generally estimated to be approximately 2 hours at 77°K). As shown, low flow rates typically have an associated relatively large polarization loss. This is because, at low flow rates, the accumulation time can be extensive and the ice "T1" then plays a larger or more dominant role. As shown, the polarization retention fraction achieved using the freezing and thawing methods is above 40% for all flow rates, and the average is about 49.9%. Therefore, as shown in **Figure 13A**, this polarization retention fraction is substantially insensitive to flow rate. The below listed data shows exemplary polarization retention fractions now achievable.

| Flow Rate | Polarization (P) _{theory} | P_{exper.} | Retention Fraction |
|---|---|---|---|
| 300 | 24 | 12.66 | 52.8% |
| 600 | 22.1 | 11.18 | 50.6% |
| 900 | 18.7 | 9.30 | 49.7% |
| 1200 | 15.9 | 7.83 | 49.2% |
| 1500 | 13.75 | 6.73 | 48.9% |
| 1800 | 12.08 | 5.90 | 48.8% |
| 2000 | 11.1 | 5.43 | 48.9% |

For example, a data point at a flow rate of 600 sccm has a theoretical polarization level of 22.1 and a corresponding experimental data point of 11.18 polarization after thaw. The initial polarization level (before- accumulation/freezing) for this flow rate is 22.1%. Therefore, the polarization retention fraction after the freeze/thaw process is 11.18/22.1 or 50.6%. Thus, advantageously, the instant thawing technique retains at least 30% of the initial polarization level and based on this data preferably above 40% of the initial polarization level, and most preferably above 45%. Further the improved retention rate increases the thawed polarization level by an order of magnitude (now reliably and predictably above about 10% in contrast to conventional thawed polarization levels of about 2%).

Although particularly suited for ¹²⁹Xe, the instant thawing method can also successfully be employed with other yperpolarized noble gases. Further, it will be appreciated by those of skill in the art, that the cryogen used to freeze the polarized gas is not limited to liquid N₂. However, if alternate refrigeration sources or cryogens are used then flow rates, accumulation rates, "warming" gas temperatures and the like should be adjusted accordingly. Further, it is desired to use refrigeration sources with temperatures at least as low as liquid nitrogen (77K) for collection of the polarized gas. Lower temperatures increase the T1 time of the solid polarized gas which results in increased relaxation times. For example, polarized gases frozen at liquid nitrogen temperatures have an ice relaxation time (T1) of approximately 2.8 hours while polarized gases frozen at liquid helium temperatures have an ice relaxation time (T1) of approximately 12 days. Therefore, in order to achieve higher polarization levels after thawing, the thawing is preferably performed within the corresponding T1 time period.

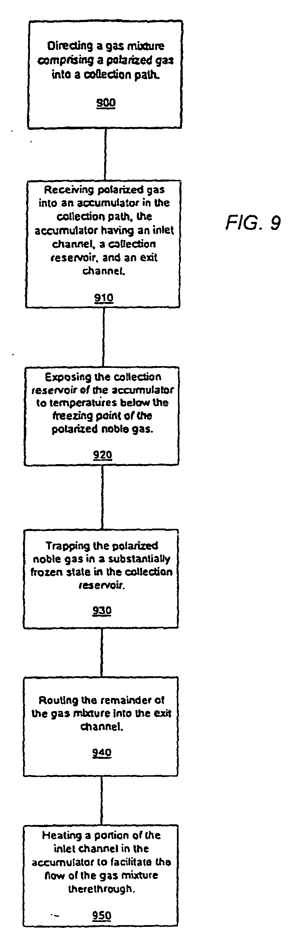

**Figures 9, 10, and 11** are block diagrams of methods associated with the instant invention. The order of the methods is not meant to be limited by the block numbers and order shown. Additional steps can also be included as operationally described hereinabove.

**Figure 9** shows steps for accumulating or collecting frozen polarized gas according to one embodiment of the instant invention A gas mixture comprising a polarized gas is directed into collection path (**Block 900**). The polarized gas is received into the accumulator in the collection path. The accumulator has an inlet channel, a collection reservoir, and an exit channel (**Block 910**). The collection reservoir is exposed to temperatures below the freezing point of the polarized noble gas (**Block 920**). The polarized gas is trapped in a substantially frozen state in the collection reservoir (preferably a total solid frozen state)(**Block 930**). The remainder of the gas mixture is routed into the exit channel (**Block 940**). A portion of the inlet channel in the accumulator is heated to facilitate the flow of the gas mixture therethrough (**Block 950**). The heating step (**Block 950**) is preferably carried out by introducing a gas separate from the gas mixture to conductively heat a predetermined area of the inlet channel, the separate gas being contained apart from the inlet and exit paths. The contained separate gas is then circulated about a portion of the inlet path to reduce the likelihood of blockage along the inlet path attributed to the exposing step.

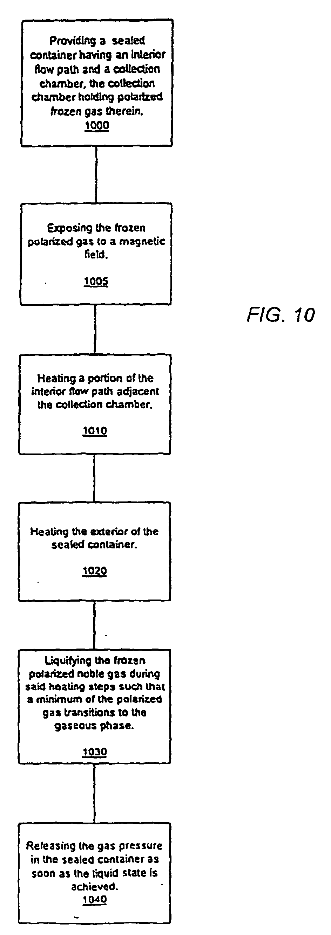

**Figure 10** illustrates a method for thawing frozen polarized gas. A sealed container is provided which includes an interior flow path and a collection chamber for holding frozen polarized gas (**Block 1000**). The frozen gas is exposed to a magnetic field (**Block 1005**). A portion of the interior flow path adjacent the collection chamber is heated (**Block 1010**). The exterior of the sealed container is also heated (**Block 1020**). The frozen gas is liquefied during the heating steps such that a minimum amount of the polarized gas transitions to the gaseous phase (and conversely, a substantial amount of the polarized gas transitions directly to the liquid phase) (**Block 1030**). Preferably, the liquefying step is carried out by closing the isolation valves and sealing the container allowing the pressure to build to a predetermined level, the level corresponding to the time it takes to provide an "instantaneous" thaw. Stated differently, the valves remain closed for as short a period as possible (as described above, less than about 10 seconds for a single patient dose), the period corresponding to the time it takes to achieve substantially full gas pressure upon opening the accumulator isolation valve. The release pressure can be calculated according to a liquid Xe vapor pressure curve. *See* V.A. Rabinovich et al., *Thermophysical Properties of Neon, Argon, Krypton, and Xenon* (Hemisphere Publishing Corp., Wash, 1988). An exemplary pressure release is thought to be less than approximately 506.625-1013.25 kPa (5-10 atm) (and at least less than about 1722.525 kPa (17 atm)) for a 0.5L accumulation in a 30cm³ accumulator at a temperature below 200K. This value will be different for different cold finger volumes, different accumulation volumes, and the temperature of the gas in liquid Xe. The Sauer et al. reference, *supra*, indicates that for Xe at 161.4K, P= 81.06 kPa (.81atm), and the triple point 289.7K, P=5775.525kPa (57atm), at 240K, P= 4053 kPa(40atm). Thus, as indicated by **Block 1040**, the gas pressure is released from the sealed container as soon as the liquid state is achieved. It is also preferred that the interior be heated as described above.

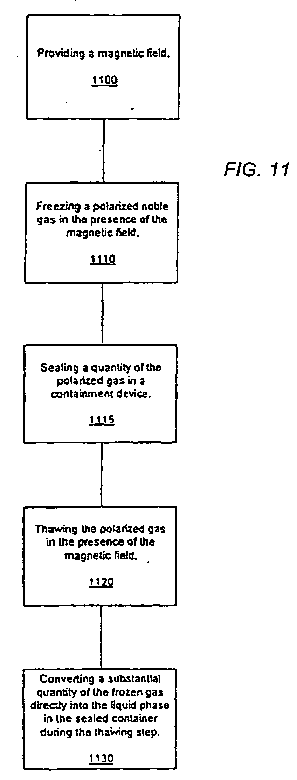

**Figure 11** illustrates a method for extending the useful polarization life of a polarized gas product. A magnetic field is provided **(Block 1100).** The polarized gas product is frozen in the presence of the magnetic field (**Block 1110)**. A quantity of the frozen polarized gas is sealed in a containment device (**Block 1115**). The polarized gas is thawed in the presence of a magnetic field (Block 1120). A substantial quantity of the frozen gas is converted directly into the liquid phase in the sealed container during the thawing step (**Block 1130**). Although not shown in this figure, various other steps can be employed along the lines described hereinabove. (For example, other steps can include, but are not limited to, decreasing the amount of ¹³¹Xe in the enriched gas mixture, heating the interior of the flow path, using a nozzle to direct the flow of gas, depressurizing the containment device by opening the valves causing the liquid to become gas and releasing the polarized gas to a interface such as a bag or other delivery device).

## Claims

1. A cryogenic accumulator **30** for collecting frozen polarized noble gases, comprising:
a primary flow channel **80** having opposing first and second ends configured to direct polarized gas therethrough;
an outer sleeve positioned **103** around said primary flow channel **80**, said outer sleeve **103** having a closed end defining a collection chamber **75** positioned below said primary flow channel **80** second end; and
a secondary flow channel **95** positioned intermediate of said primary flow channel **80** and said outer sleeve **103**, said secondary flow channel **95** having a closed end positioned in close proximity to said primary flow channel second end.

2. A cryogenic accumulator **30** according to Claim 1, said secondary flow channel **95** having a cylindrically shaped inner wall **93a,** wherein said inner wall **93a** defines said primary flow channel **80**.

3. A cryogenic accumulator **30** according to Claim 1 or 2, said secondary flow channel **95** having an outer wall **93b**, wherein said outer wall **93b** and said outer sleeve **103** define a buffer gas exit channel **90** therebetween.

4. A cryogenic accumulator **30** according to any of Claims 1 to 3, wherein said primary flow channel second end is a nozzle **110**.

5. A cryogenic accumulator **30** according to Claim 3, further comprising first and second isolation valves **35**, **37** in communication with said primary flow channel **80** and said buffer gas exit channel **90**.

6. A cryogenic accumulator **30** according to Claim 5, wherein said first isolation valve **35** is positioned at the first end of said primary flow channel to control the flow of a target gas therethrough.

7. A cryogenic accumulator **30** according to Claim 5, wherein said second isolation valve **37** is positioned spaced-apart from said outer sleeve **103** closed end along said buffer gas exit channel **90** to releasably seal and control the release of gas therethrough.

8. A cryogenic accumulator **30** according to any of Claims 1 to 7, further comprising a secondary flow channel inlet **125** and vent port **126**, and a conduit **145** in fluid communication with said inlet **125**, wherein said conduit **145** extends along a major portion of said secondary flow channel **95** to thereby direct the flow of a warming gas to a predetermined area of said primary flow channel **80**.

9. A cryogenic accumulator **30** according to any of Claims 1 to 8, in combination with a hyperpolarizer unit **10**, wherein said accumulator **30** is configured to detachably release from said hyperpolarizer unit **10**.

10. A cryogenic accumulator **30** according to any of Claims 1 to 9, wherein said secondary flow channel **95** is separate from said primary flow channel **80**.

11. A cryogenic accumulator **30** according to any of Claims 1 to 10, wherein said secondary flow channel **95** is a heating jacket **93** configured to circulate a gas therethrough.

12. A cryogenic accumulator **30** according to any of Claims 1 to 11, wherein said secondary flow channel **95** includes a longitudinally extending conduit **145** configured to direct a gas to said secondary flow channel **95** closed end adjacent said second end of said primary flow channel **80**.

13. A cryogenic accumulator **30** according to any of Claims 1 to 12, further comprising at least one permanent field magnet **40** positioned adjacent said collection chamber **75.**

14. A cryogenic accumulator **30** according to any of Claims 1 to 13, further comprising a cryogenic refrigeration source **42** operably associated with said collection chamber **75**.

15. A cryogenic accumulator **30** according to any of Claims 1 to 14, wherein said secondary flow channel **95** is configured to circulate ambient temperature gaseous nitrogen therethrough.

16. A cryogenic accumulator **30** according to any of Claims 1 to 15, said accumulator **30** further comprising at least one secondary flow channel vent **126** in communication with said secondary flow channel **95**.

17. A cryogenic accumulator **30** according to Claim 16, further comprising a flow adjustment valve operably associated with said vent **126** to adjust the flow of a gas therein thereby adjusting the heat supplied to at least a portion of said primary flow channel **80**.

18. A cryogenic accumulator **30** according to any of Claims 1 to 17, wherein said outer sleeve **103**, said secondary flow channel **95**, and said primary flow channel **80** are radially aligned along a major portion of the length of said accumulator **30**.

19. A cryogenic accumulator according to any of Claims 1 to 18, wherein said second end is configured as a flow nozzle **110**, and wherein said flow nozzle **110** is aligned with and positioned adjacent to said collection chamber **75**.

20. A cryogenic accumulator **30** according to Claim 19, further comprising a heat source 93 positioned intermediate of said primary flow channel **80** and said outer sleeve **103** before said collection chamber **75**, said heat source arranged to provide heat to said flow nozzle **110**.

21. A cryogenic accumulator **30** according to Claim 20, wherein said heat source **93** includes a conduit **145** for directing a flow of a predetermined gas to said flow nozzle **110.**

22. A method for collecting frozen noble polarised gas, comprising the steps of:
directing a gas mixture comprising a polarized noble gas and a second gas along a collection path and into an accumulator **30**;
receiving the gas mixture into the accumulator **30** positioned in the collection path, the accumulator having an inlet channel **80**, a collection reservoir **75**, and an exit channel **90**;
cooling the collection reservoir **75** to temperatures below the freezing point of the polarized noble gas;
capturing polarized noble gas in a substantially frozen state **100** in the collection reservoir **75**;
passing the remainder of the gas mixture including the second gas into the exit channel **90**; and
heating a portion of the inlet channel **80** in the accumulator **30** to facilitate the flow of the gas mixture therethrough.

23. A method according to Claim 22, wherein said heating step comprises the steps of:
introducing a gas separate from the gas mixture to heat a predetermined area of the inlet channel **80,** the separate gas being contained apart **95** from the inlet **80** and exit paths **90**, and the collection reservoir **75**;
circulating the gas separate from the gas mixture about a portion of the inlet path **80** to provide conductive heat to selected portions of the inlet path **80** and reduce the likelihood of blockage along the inlet path **80** attributed to said cooling step.

24. A method according to Claim 22 or Claim 23, wherein said directing step includes flowing the gas mixture through a directional nozzle **110** into the collection reservoir **75**.

25. A method according to any of Claims 22 to 24, wherein said cooling step includes immersing the bottom reservoir **75** into a liquid cryogen bath **43.**

26. A method according to any of Claims 22 to 25, wherein said heating step is provided by the steps of:
circulating room temperature nitrogen gas around the outside of at least a portion of the inlet channel **80**; and
capturing the nitrogen gas and venting it to atmosphere away from the frozen accumulated noble gas **100.**

27. A method according to any of Claims 22 to 26, further comprising the steps of:
accumulating polarized noble gas **100** in the collection reservoir **75**; and
exposing the gas to a magnetic field during accumulation.

28. A method according to Claim 27, further comprising the steps of:
detaching the accumulator **30** from a portion of the collection path and
transporting the accumulator **30** with frozen polarized gas **100** in the presence of a magnetic field to a remote site.

29. A method according to any of Claims 22 to 28, wherein said polarized gas is ¹²⁹Xe.

## Patentansprüche

1. Tieftemperaturspeicher 30 zum Aufnehmen von gefrorenen polarisierten Edelgasen umfassend:
einen ersten Strömungsweg 80 mit einem ersten und einem zweiten Ende, die sich gegenüberliegen, der für die Durchleitung von polarisiertem Gas ausgelegt ist;
eine äußere Umhüllung 103, die um den genannten ersten Strömungsweg 80 angeordnet ist, wobei die genannte äußere Umhüllung 103 ein geschlossenes Ende aufweist, das eine Auffangkammer 75 bildet, die unterhalb des genannten zweiten Endes des ersten Strömungsweges 80 angeordnet ist; und
einen zweiten Strömungsweg 95, der zwischen dem genannten ersten Strömungsweg 80 und der genannten äußeren Umhüllung 103 angeordnet ist, wobei der genannte zweite Strömungsweg 95 ein geschlossenes Ende aufweist, das in nächster Nähe des genannten zweiten Endes des ersten Strömungsweges angeordnet ist.

2. Tieftemperaturspeicher 30 gemäß Anspruch 1, worin der genannte zweite Strömungsweg 95 eine zylindrisch geformte Innenwand 93a aufweist und die genannte Innenwand 93a den genannten ersten Strömungsweg 80 bildet.

3. Tieftemperaturspeicher 30 gemäß Anspruch 1 oder 2, worin der genannte zweite Strömungsweg 95 eine Außenwand 93b aufweist und zwischen der genannten Außenwand 93b und der genannten äußeren Umhüllung 103 ein Austrittsweg für das Puffergas 90 gebildet ist.

4. Tieftemperaturspeicher 30 gemäß einem der Ansprüche 1 bis 3, worin das genannte zweite Ende des ersten Strömungsweges eine Düse 110 ist.

5. Tieftemperaturspeicher 30 gemäß Anspruch 3, der weiterhin ein erstes und ein zweites Absperrventil 35, 37 umfaßt, die mit dem genannten ersten Strömungsweg 80 und dem genannten Austrittsweg für das Puffergas 90 in Verbindung stehen.

6. Tieftemperaturspeicher 30 gemäß Anspruch 5, worin das genannte erste Absperrventil 35 am ersten Ende des genannten ersten Strömungsweges angeordnet ist, um den Durchfluß eines bestimmungsgemäßen Gases zu steuern.

7. Tieftemperaturspeicher 30 gemäß Anspruch 5, worin das genannte zweite Absperrventil 37 in einigem Abstand von dem genannten geschlossenen Ende der äußeren Umhüllung 103 am genannten Austrittsweg für das Puffergas 90 angeordnet ist, um den Austritt des durchfließenden Gases zu unterbinden und zu steuern.

8. Tieftemperaturspeicher 30 gemäß einem der Ansprüche 1 bis 7, der weiterhin einen Einlaß 125 und eine Auslaßöffnung 126 für den zweiten Strömungsweg und eine Leitung 145 in fließtechnischer Verbindung mit dem genannten Einlaß 125 umfaßt, worin sich die genannte Leitung 145 über einen großen Teil des genannten zweiten Strömungsweges 95 erstreckt, wodurch ein wärmender Gasstrom an eine bestimmte Stelle des genannten ersten Strömungsweges 80 geleitet wird.

9. Tieftemperaturspeicher 30 gemäß einem der Ansprüche 1 bis 8 in Kombination mit einer Hyperpolarisator-Einheit 10, worin der genannte Speicher 30 abtrennbar mit der genannten Hyperpolarisator-Einheit 10 verbunden ist.

10. Tieftemperaturspeicher 30 gemäß einem der Ansprüche 1 bis 9, worin der genannte zweite Strömungsweg 95 von dem genannten ersten Strömungsweg 80 getrennt ist.

11. Tieftemperaturspeicher 30 gemäß einem der Ansprüche 1 bis 10, worin der genannte zweite Strömungsweg 95 einen Heizmantel 93 darstellt, der für den Durchfluß eines Gases ausgelegt ist.

12. Tieftemperaturspeicher 30 gemäß einem der Ansprüche 1 bis 11, worin der genannte zweite Strömungsweg 95 eine in Längsrichtung verlaufende Leitung 145 einschließt, die so aufgebaut ist, daß ein Gas zu dem genannten geschlossenen Ende des zweiten Strömungsweges 95 geleitet wird, das sich in der Nähe des genannten zweiten Endes des genannten ersten Strömungsweges 80 befindet.

13. Tieftemperaturspeicher 30 gemäß einem der Ansprüche 1 bis 12, der weiterhin mindestens einen Permanentmagneten 40 umfaßt, der in der Nähe der genannten Auffangkammer 75 angeordnet ist.

14. Tieftemperaturspeicher 30 gemäß einem der Ansprüche 1 bis 13, der weiterhin eine Tiefkühlvorrichtung 42 umfaßt, die mit der genannten Auffangkammer 75 funktionell verbunden ist.

15. Tieftemperaturspeicher 30 gemäß einem der Ansprüche 1 bis 14, worin der genannte zweite Strömungsweg 95 für den Durchfluß von gasförmigem Stickstoff, der Umgebungstemperatur aufweist, ausgelegt ist.

16. Tieftemperaturspeicher 30 gemäß einem der Ansprüche 1 bis 15, worin der genannte Speicher 30 weiterhin mindestens einen Auslaß für den zweiten Strömungsweg 126 umfaßt, der mit dem genannten zweiten Strömungsweg 95 in Verbindung steht.

17. Tieftemperaturspeicher 30 gemäß Anspruch 16, der weiterhin einen Durchflußregler umfaßt, der mit dem genannten Auslaß 126 funktionell verbunden ist, um den durchfließenden Gasstrom einzustellen, wodurch die Wärme, die auf mindestens einen Abschnitt des genannten ersten Strömungsweges 80 einwirkt, reguliert wird.

18. Tieftemperaturspeicher 30 gemäß einem der Ansprüche 1 bis 17, worin die genannte äußere Umhüllung 103, der genannte zweite Strömungsweg 95 und der genannte erste Strömungsweg 80 über einen großen Teil der Länge des genannten Speichers 30 radial ausgerichtet sind.

19. Tieftemperaturspeicher 30 gemäß einem der Ansprüche 1 bis 18, worin das genannte zweite Ende als Strömungsdüse 110 ausgebildet ist und worin die genannte Strömungsdüse 110 auf die genannte Auffangkammer 75 ausgerichtet und in ihrer Nähe angeordnet ist.

20. Tieftemperaturspeicher 30 gemäß Anspruch 19, der weiterhin eine Heizquelle 93 umfaßt, die zwischen dem genannten ersten Strömungsweg 80 und der genannten äußeren Umhüllung 103 vor der genannten Auffangkammer 75 angeordnet ist, wobei die genannte Heizquelle so angeordnet ist, daß sie die genannte Strömungsdüse 110 erwärmt.

21. Tieftemperaturspeicher 30 gemäß Anspruch 20, worin die genannte Heizquelle 93 eine Leitung 145 einschließt, die einen Strom eines bestimmten Gases auf die genannte Strömungsdüse 110 lenkt.

22. Verfahren zum Aufnehmen von gefrorenem polarisierten Edelgas umfassend die Schritte:
Leiten einer Gasmischung, die ein polarisiertes Edelgas und ein zweites Gas umfaßt, entlang eines Aufnahmeweges und in einen Speicher 30;
Auffangen der Gasmischung im Speicher 30, der im Aufnahmeweg angeordnet ist, wobei der Speicher einen Einlaßweg 80, einen Auffangbehälter 75 und einen Austrittsweg 90 aufweist;
Kühlen des Auffangbehälters 75 auf Temperaturen unterhalb des Gefrierpunktes des polarisierten Edelgases;
Einschließen des polarisierten Edelgases in einem im Wesentlichen gefrorenen Zustand 100 im Auffangbehälter 75;
Weiterleiten des restlichen Teils der Gasmischung, der das zweite Gas einschließt, in den Austrittsweg 90; und
Erwärmen eines Abschnittes des Einlaßweges 80 im Speicher 30, um den Durchfluß der durchgeleiteten Gasmischung zu erleichtern.

23. Verfahren gemäß Anspruch 22, worin der genannte Erwärmungsschritt die folgenden Schritte umfaßt:
Einleiten eines von der Gasmischung gesonderten Gases, um eine bestimmte Stelle des Einlaßweges BO zu erwärmen, wobei das gesonderte Gas 95 außerhalb des Einlaßweges 80, des Austrittsweges 90 und des Auffangbehälters 75 vorliegt;
Führen des von der Gasmischung gesonderten Gases auf einen Abschnitt des Einlaßweges 80, um Ableitungswärme auf ausgewählte Abschnitte des Einlaßweges 80 zu leiten und die Wahrscheinlichkeit einer Blockierung innerhalb des Einlaßweges 80, die auf dem genannten Kühlschritt beruht, zu verringern.

24. Verfahren gemäß Anspruch 22 oder 23, worin der genannte Schritt des Leitens das Einleiten der Gasmischung durch eine gerichtete Düse 110 in den Auffangbehälter 75 einschließt.

25. Verfahren gemäß einem der Ansprüche 22 bis 24, worin der genannte Kühlschritt das Eintauchen des unteren Behälters 75 in ein flüssiges Tiefkühlbad 43 einschließt.

26. Verfahren gemäß einem der Ansprüche 22 bis 25, worin der genannte Erwärmungsschritt durch die folgenden Schritte verwirklicht wird:
Leiten von Stickstoffgas, das Raumtemperatur aufweist, um die Außenseite mindestens eines Abschnittes des Einlaßweges 80; und
Auffangen des Stickstoffgases und dessen Ausleiten in die Atmosphäre und wegführend von dem gespeicherten gefrorenen Edelgas 100.

27. Verfahren gemäß einem der Ansprüche 22 bis 26 umfassend weiterhin die Schritte:
Speichern des polarisierten Edelgases 100 im Auffangbehälter 75; und
Einbringen des Gases in ein magnetisches Feld während des Speicherns.

28. Verfahren gemäß Anspruch 27, umfassend weiterhin die Schritte:
Abtrennen des Speichers 30 von einem Abschnitt des Aufnahmeweges und
Transportieren des Speichers 30 mit gefrorenem polarisierten Gas 100 in Gegenwart eines magnetischen Feldes zu einer entfernten Stelle.

29. Verfahren gemäß einem der Ansprüche 22 bis 28, worin das genannte polarisierte Gas ¹²⁹Xe ist.

## Revendications

1. Accumulateur cryogénique (30) destiné à collecter des gaz nobles polarisés gelés, comprenant :
un canal d'écoulement primaire (80) présentant des première et seconde extrémités opposées, configuré de manière à diriger un gaz polarisé à travers ;
un manchon externe (103) positionné autour dudit canal d'écoulement primaire (80), ledit manchon externe (103) présentant une extrémité fermée définissant une chambre de collecte (75) positionnée au-dessous de ladite seconde extrémité du canal d'écoulement primaire (80) ; et
un canal d'écoulement secondaire (95) positionné de manière intermédiaire par rapport audit canal d'écoulement primaire (80) et audit manchon externe (103), ledit canal d'écoulement secondaire (95) présentant une extrémité fermée positionnée en étroite proximité par rapport à ladite seconde extrémité du canal d'écoulement primaire.

2. Accumulateur cryogénique (30) selon la revendication 1, ledit canal d'écoulement secondaire (95) présentant une paroi interne formée de manière cylindrique (93a), dans lequel ladite paroi interne (93a) définit ledit canal d'écoulement primaire (80).

3. Accumulateur cryogénique (30) selon la revendication 1 ou 2, ledit canal d'écoulement secondaire (95) présentant une paroi externe (93b), dans lequel ladite paroi externe (93b) et ledit manchon externe (103) définissent un canal de sortie de gaz tampon (90) entre eux.

4. Accumulateur cryogénique (30) selon l'une quelconque des revendications 1 à 3, dans lequel ladite seconde extrémité de canal d'écoulement primaire est un injecteur (110).

5. Accumulateur cryogénique (30) selon la revendication 3, comprenant, en outre, des première et seconde vannes d'isolement (35, 37) en communication avec ledit canal d'écoulement primaire (80) et ledit canal de sortie de gaz tampon (90).

6. Accumulateur cryogénique (30) selon la revendication 5, dans lequel ladite première vanne d'isolement (35) est positionnée au niveau de la première extrémité dudit canal d'écoulement primaire afin de commander l'écoulement d'un gaz cible à travers.

7. Accumulateur cryogénique (30) selon la revendication 5, dans lequel ladite seconde vanne d'isolement (37) est positionnée de manière séparée par rapport à l'extrémité fermée dudit manchon externe (103) le long dudit canal de sortie de gaz tampon (90) afin d'assurer l'étanchéité de manière réversible et la commande de la libération du gaz à travers.

8. Accumulateur cryogénique (30) selon l'une quelconque des revendications 1 à 7, comprenant, en outre, une entrée de canal d'écoulement secondaire (125) et un orifice de mise à l'air (126) et un conduit (145) en communication fluidique avec ladite entrée (125), dans lequel ledit conduit (145) s'étend le long d'une partie principale dudit canal d'écoulement secondaire (95) afin de diriger ainsi l'écoulement d'un gaz chaud vers une zone prédéterminée dudit canal d'écoulement primaire (80).

9. Accumulateur cryogénique (30) selon l'une quelconque des revendications 1 à 8, en association avec une unité fortement polarisante (10) dans lequel ledit accumulateur (30) est configuré afin de se libérer de manière amovible de ladite unité fortement polarisante (10).

10. Accumulateur cryogénique (30) selon l'une quelconque des revendications 1 à 9, dans lequel ledit canal d'écoulement secondaire (95) est séparé par rapport audit canal d'écoulement primaire (80).

11. Accumulateur cryogénique (30) selon l'une quelconque des revendications 1 à 10, dans lequel ledit canal d'écoulement secondaire (95) est une double enveloppe chauffante (93) configurée de manière à faire circuler un gaz à travers.

12. Accumulateur cryogénique (30) selon l'une quelconque des revendications 1 à 11, dans lequel ledit canal d'écoulement secondaire (95) comporte un conduit s'étendant longitudinalement (145) configuré de manière à diriger un gaz vers ladite extrémité fermée du canal d'écoulement secondaire (95) de manière adjacente à ladite seconde extrémité dudit canal d'écoulement primaire (80).

13. Accumulateur cryogénique (30) selon l'une quelconque des revendications 1 à 12, comprenant, en outre, au moins un aimant permanent (40) positionné de manière adjacente à ladite chambre de collecte (75).

14. Accumulateur cryogénique (30) selon l'une quelconque des revendications 1 à 13, comprenant, en outre, une source de réfrigération cryogénique (42) associée de manière opérationnelle à ladite chambre de collecte (75).

15. Accumulateur cryogénique (30) selon l'une quelconque des revendications 1 à 14, dans lequel ledit canal d'écoulement secondaire (95) est configuré de manière à faire circuler de l'azote gazeux à température ambiante à travers.

16. Accumulateur cryogénique (30) selon l'une quelconque des revendications 1 à 15, ledit accumulateur (30) comprenant, en outre, au moins une mise à l'air de canal d'écoulement secondaire (126) en communication avec ledit canal d'écoulement secondaire (95)

17. Accumulateur cryogénique (30) selon la revendication 16, comprenant, en outre, une vanne de réglage d'écoulement associée de manière opérationnelle à ladite mise à l'air (126) afin de régler le débit d'un gaz à l'intérieur réglant ainsi la chaleur délivrée à au moins une partie dudit canal d'écoulement primaire (80).

18. Accumulateur cryogénique (30) selon l'une quelconque des revendications 1 à 17, dans lequel ledit manchon interne (103), ledit canal d'écoulement secondaire (95) et ledit canal d'écoulement primaire (80) sont alignés radialement suivant une partie principale de la longueur dudit accumulateur (30).

19. Accumulateur cryogénique (30) selon l'une quelconque des revendications 1 à 18, dans lequel ladite seconde extrémité est configurée comme un injecteur d'écoulement (110) et dans lequel ledit injecteur d'écoulement (110) est aligné avec ladite chambre de collecte (75) et positionné de manière adjacente à celle-ci.

20. Accumulateur cryogénique (30) selon la revendication 19, comprenant, en outre, une source de chaleur (93) positionnée de manière intermédiaire par rapport audit canal d'écoulement primaire (80) et audit manchon externe (103) avant ladite chambre de collecte (75), ladite source de chaleur étant agencée afin de fournir de la chaleur audit injecteur d'écoulement (110).

21. Accumulateur cryogénique (30) selon la revendication 20, dans lequel ladite source de chaleur (93) comporte un conduit (145) destiné à acheminer un débit d'un gaz prédéterminé vers ledit injecteur d'écoulement (110).

22. Procédé de collecte de gaz polarisé noble gelé, comprenant les étapes de :
acheminement d'un mélange gazeux comprenant un gaz noble polarisé et un second gaz le long d'un trajet de collecte et vers un accumulateur (30) ;
réception du mélange gazeux dans l'accumulateur (30) positionné sur le trajet de collecte, l'accumulateur comportant un canal d'entrée (80), un réservoir de collecte (75) et un canal de sortie (90) ;
refroidissement du réservoir de collecte (75) à une température inférieure au point de congélation du gaz noble polarisé ;
capture du gaz noble polarisé dans un état sensiblement gelé (100) dans le réservoir de collecte (75) ;
transfert du reste du mélange gazeux, comprenant le second gaz, dans le canal de sortie (90) ; et
chauffage d'une partie du canal d'entrée (80) dans l'accumulateur (30) afin de faciliter l'écoulement du mélange gazeux à travers.

23. Procédé selon la revendication 22, dans lequel ladite étape de chauffage comprend les étapes de :
introduction d'un gaz distinct du mélange gazeux afin de chauffer une zone prédéterminée du canal d'entrée (80), le gaz distinct étant contenu à part (95) par rapport aux trajets d'entrée (80) et de sortie (90) et au réservoir de collecte (75) ;
mise en circulation du gaz distinct du mélange gazeux autour d'une partie du trajet d'entrée (80) afin d'assurer un chauffage par conduction vers des parties sélectionnées du trajet d'entrée (80) et de réduire le risque de blocage le long du trajet d'entrée (80) attribué à ladite étape de refroidissement.

24. Procédé selon la revendication 22 ou la revendication 23, dans lequel ladite étape d'acheminement comporte la mise en circulation du mélange gazeux à travers un injecteur directionnel (110) dans le réservoir de collecte (75).

25. Procédé selon l'une quelconque des revendications 22 à 24, dans lequel ladite étape de refroidissement comporte l'immersion du fond de réservoir (75) dans un bain cryogénique liquide (43).

26. Procédé selon l'une quelconque des revendications 22 à 25, dans lequel ladite étape de chauffage est assurée par les étapes de :
mise en circulation d'azote gazeux à température ambiante autour de l'extérieur d'au moins une partie du canal d'entrée (80) ; et
capture de l'azote gazeux et sa mise à l'atmosphère isolé du gaz noble accumulé gelé (100).

27. Procédé selon l'une quelconque des revendications 22 à 26, comprenant, en outre, les étapes de :
accumulation de gaz noble polarisé (100) dans le réservoir de collecte (75) ; et
exposition du gaz à un champ magnétique au cours de l'accumulation.

28. Procédé selon la revendication 27, comprenant, en outre, les étapes de :
séparation de l'accumulateur (30) d'une partie du trajet de collecte ; et
transport de l'accumulateur (30) avec le gaz polarisé gelé (100) en présence d'un champ magnétique vers un site distant.

29. Procédé selon l'une quelconque des revendications 22 à 28, dans lequel ledit gaz polarisé est du ¹²⁹Xe.
